# EUROPÄISCHE PATENTANMELDUNG

(11) **EP 4 062 857 A1**
(43) Veröffentlichungstag der Anmeldung: **28.09.2022**
(21) Anmeldenummer: 22163974.3
(22) Anmeldetag: 24.03.2022
(51) Int. Cl.: A61B 46/20, A61B 46/23, A61M 16/06

(54) **ANÄSTHESIESCHUTZBAND UND ANLEGEVERFAHREN**

(30) Priorität: 25.03.2021 CH 3192021
(71) Anmelder: Hartmann-Forth, Thomas, 5054 Kirchleerau (CH)
(72) Erfinder: Hartmann-Forth, Thomas, 5054 Kirchleerau (CH)
(74) Vertreter: Prins Intellectual Property AG

(57) **Zusammenfassung**

Bei einer Anästhesieschutzvorrichtung (0) für einen Patienten, welche vor einem operativen Eingriff appliziert wird, wobei die Anästhesieschutzvorrichtung (0) von einem einstückigen teilweise mehrlagigen und teilweise gepolsterten Textil (0) mit einer Innenfläche (I) gebildet ist, soll eine vereinfachte flexible Applizierung ermöglicht sein. Dies wird dadurch erreicht, dass die Anästhesieschutzvorrichtung (0) als offenes, teilweise mehrlagiges und gepolstert ausgeführtes textiles Anästhesieschutzband (0) ausgestaltet ist, welche mit einem Zentralauflageabschnitt (1), einem ersten Schutzabschnitt (2) mit mindestens einem Schutzpolster (20) zwischen zwei textilen Lagen des ersten Schutzabschnitts (2) und einem zweiten Schutzabschnitt (3) ausgestattet ist, sodass nach dem Umschlagen des Anästhesieschutzbandes (0) um einen Körperteil eine lösbare Verbindung mittels erster Bandverschlussmittel (22) und zweiter Bandverschlussmittel (30) ausbildbar ist.

## Beschreibung

### Technisches Gebiet

Die vorliegende Erfindung beschreibt eine Anästhesieschutzvorrichtung für einen Patienten, welche vor einem operativen Eingriff appliziert wird, wobei dieAnästhesieschutzvorrichtung von einem einstückigen teilweise mehrlagigen und teilweise gepolsterten Textil mit einer Innenfläche gebildet ist, wobei die Anästhesieschutzvorrichtung derartum einen Körperteil des Patienten derart legbar ist, die Innenfläche mindestens teilweise direkt auf der Haut des Patienten aufliegt und die Anästhesieschutzvorrichtung lösbar am Patienten befestigbar ist, sowie ein Verfahren zum Anlegen eines mehrlagigen, teilweise gepolsterten textilen Anästhesieschutzbandes.

### Stand der Technik

Vorrichtungen zur Atemwegssicherung bzw. allgemeinen Patientensicherung in der Anästhesie, sind bereits bekannt.

Zum Schutz des Kopfes, beispielsweise während einer Schilddrüsenoperation, von Patienten, werden heute immernoch aufwändige Wickel aus Gaze und Verbandsmaterial vor jeder Operation individuell angefertigt und dem Patienten nach der Narkoseeinleitung und Intubation angelegt. Dieser Vorgang muss vor Beginn des chirurgischen Eingriffs nach Intubation und Installation von Beatmungsschläuchen durchgeführt werden, wobei der Patient einen Kopfverband oder Kopfwickel erhält, dabei muss eine Beatmung und Überwachung des Patienten erlaubt bleiben. Versorgungsschläuche und Kabel werden zum Kopf und in die Mund- und/oder Nasenhöhle geführt verlegt.

Vor allem Augen- und Nasenbereich müssen derart präpariert werden, dass eine ausreichende Beatmung möglich ist und der Chirurg sogar gefahrlos seine Finger, Hände oder sogar Unterarme am oder auf dem Kopf des Patienten während der Operation ablegen kann. Diese klassischen Einwegkopfverbände müssen vor der Operation an den Kopf des Patienten angepasst werden und nach Beendigung der Operation wieder entfernt werden. Gleichwohl muss die schnellstmögliche Entfernung während eines intraoperativen Notfalls gewährleistet sein. Der angepasste Kopfwickel ist jeweils eine Spezialanfertigung. Das Resultat hängt vom Können des OP-Personals ab, ist zeitaufwändig in der Anpassung und die erreichten Ergebnisse sind oft sehr unterschiedlich, da es einen grossen Herstellungsspielraum gibt.

In der WO2011019495 ist ein Anästhesieschutz gezeigt, welcher aus einer schaumartigen Lage gebildet ist, welche um den Kopf eines Patienten gelegt werden kann. Dabei sind Aussparungen in die Anästhesieschutzvorrichtung eingebracht, sodass Augen, Mund und/oder Nase von aussen zugänglich sind, der Kopf aber ansonsten abgedeckt ist. Die beschriebene Anästhesieschutzvorrichtung wird vor der Operation um den Kopf des Patienten gelegt und verschlossen, wobei Verschlussmittel, wie ein Reissverschluss oder Klebestreifen verwendet werden. Damit wird die ein- oder mehrlagige Schaumlage am Kopf lösbar befestigt. Die vor allem zu schützenden Bereiche, wie der Augenbereich, sind hier aber meist freigelassen, was nachteilig ist. Diese Bereiche müssten entsprechend mit zusätzlichen Vorrichtungen verdeckt werden, was zu Mehraufwand führt. Die Fixierung der Anästhesieschutzvorrichtung aus einer Kunststoffschaumlage ist aufwändig und erfolgt über eine Vielzahl von Verschlussmitteln, die am Rand der Kunststoffschaumlage verteilt sind.

Nachteilig an dieser Anästhesieschutzvorrichtung ist ausserdem, dass die Kunststoffschaumlage teilweise weit von den Konturen des Kopfes beabstandet bleibt. Die Kunststoffschaumlage oder Kunststofflage ist zwar flexibel, aber da die Dicke ausreichend dick gewählt werden muss, um Gesichtsbereiche gegen mechanische Einwirkungen zu schützen, wird eine röhrenförmige Hülle um den Kopf des Patienten erreicht. Folglich gibt es Bereiche der Anästhesieschutzvorrichtung, welche sehr weit von der Oberfläche des Kopfes des Patienten entfernt sind. Eine solche Anästhesieschutzvorrichtung ist nur unzureichend auf verschiedene Kopfgrössen anpassbar. Es müssen in jedem Fall für unterschiedlich gross Köpfe unterschiedlich dimensionierte Kunststoffschaumlagen vorhanden sein. Das OP-Personal ist damit sehr unflexibel mit der Anpassung unterschiedlicher Kunststoffschaumlagen an unterschiedliche Patienten.

Aus der EP3689408 ist eine textile gepolsterte mehrteilige Anästhesieschutzvorrichtung bekannt, welche als Strumpf oder Schlauch ausgestaltet ist und über den Kopf eines Patienten gezogen angelegt wird. Durch vorgesehene Befestigungsmittel wird der Strumpf am Kopf des Patienten fixiert, ist also einfach lösbar am Kopf befestigbar. Mund- und Nasenausschnitte, sowie Polster an den späteren Positionen der Augen müssen in auf den Patienten abgestimmter strumpfartiger Anästhesieschutzvorrichtung passend angeordnet sein, sodass nach dem über den Kopf ziehen der Anästhesieschutzvorrichtung die Schutzpolster und Aussparungen an den gewünschten Stellen zu liegen kommen. Nebst dem Nachteil, dass die Anordnung der Aussparungen und Schutzpolster mehrere Grössen der strumpfartigen Anästhesieschutzvorrichtung bedingt, ist der Vorgang des Anlegens der Anästhesieschutzvorrichtung noch nicht optimal. Zuerst muss die ausreichend grosse Anästhesieschutzvorrichtung gewählt werden, wobei die Position der Schutzpolster nicht immer stimmt, wonach anschliessend der Kopf des Patienten längere Zeit angehoben und gehalten werden muss, bis die strumpfartige Struktur über den Kopf bis zum Hals gezogen worden ist.

Auch, wenn derartige Anästhesieschutzvorrichtungen neben Schilddrüsenoperationen bei anderen operativen chirurgischen Eingriffen genutzt werden, ergeben sich die gleichen zu lösenden Nachteile, dass immer mehrere Grössen der Anästhesieschutzvorrichtung auf Patienten abgestimmt vorhanden sein müssen und das das Anlegen am Patienten immernoch problematisch ist.

### Darstellung der Erfindung

Die vorliegende Erfindung hat sich zur Aufgabe gestellt eine Anästhesieschutzvorrichtung zu schaffen, welche in einer Grösse, in unisex-Ausführung und für Erwachsene und Kinder einsetzbar vorliegt und im Vergleich zum Stand der Technik einfacher und schneller anlegbar oder anziehbar ist.

Die Anästhesieschutzvorrichtung soll den gewohnten Schutz liefern, Augen, Mund und Nase des Patienten schützen, Schläuche und Kabel während der Operation geführt halten, während der Operation den Operateur nicht stören bzw. sogar die Ablage von Operationswerkzeug oder das Aufstützen erlauben und besonders einfach vor der Operation angelegt und nach der Operation entfernt werden können.

Zur Lösung dieser Aufgabe wird eine Anästhesieschutzvorrichtung als mehrlagiges teilweise gepolstertes Textil in Form eines Gesichtsbandes mit drei Abschnitten nach Patentanspruch 1 eingeführt.

Weitere vorteilhafte Abwandlungen werden von den abhängigen Patentansprüchen beansprucht.

### Kurze Beschreibung der Zeichnungen

Einzelheiten und Vorzüge der Erfindung ergeben sich aus der nachfolgenden Beschreibung auf Basis der beiliegenden Figuren, wobei Variationen von Merkmalskombinationen in die abhängigen Patentansprüche aufgenommen wurden.
- Figur 1a: zeigt eine schematische Aufsicht auf eine Innenfläche einer mehrlagigen textilen Anästhesieschutzvorrichtung vor der Applizierung, während
- Figur 1b: eine schematische Aufsicht auf die Aussenfläche der mehrlagigen textilen Anästhesieschutzvorrichtung gemäss Figur 1a vor der Applizierung zeigt.
- Figur 2a: zeigt eine Aufsicht auf die Innenfläche der mehrlagigen textilen Anästhesieschutzvorrichtung, untergeschoben unter einen Kopf eines Patienten als erster Schritt des Anlegeverfahrens der Anästhesieschutzvorrichtung, bevor Schläuche und Kabel platziert werden, während
- Figur 2b: eine perspektivische Ansicht einer teilweise um den Kopf des Patienten umgelegten und befestigten Anästhesieschutzvorrichtung zeigt, wobei auch Schläuche und Kabel bereits fixiert sind und
- Figur 2c und 2d: perspektivische Ansichten der vollständig umgelegten und mit entsprechenden Mitteln befestigten Anästhesieschutzvorrichtung mit fixierten und gesichert geführten Schläuchen und Kabeln zeigt.

### Beschreibung

Hier wird eine Anästhesieschutzvorrichtung 0 als Anästhesieschutzband 0 ausgeführt, vorgestellt, wobei das gesamte Anästhesieschutzband 0 als teilweise mehrlagiges, teilweise gepolstertes Textil mit mehreren Abschnitten entlang seiner Längsausdehnung ausgestaltet ist. Wenn dieses Anästhesieschutzband 0 beispielsweise bei einem operativen Eingriff am Kopf K oder Hals eines Patienten eingesetzt wird, kann man es auch als Gesichtschutzband 0 bezeichnen. Der Einsatzzweck sind aber sämtliche Operationen, die an einem Körperteil eines Patienten durchgeführt werden sollen, bei welchen das Anästhesieschutzband 0 jeweils vorgängig um den Körperteil umschlagbar lösbar angeordnet werden kann und auch für die Führung und Halterung von Versorgungsschläuchen und Kabeln dient. Entsprechend ist das Anästhesieschutzband 0 als offenes Anästhesieschutzband 0 ausgeführt und nicht als Endlosband und ist auch nicht strumpfförmig, da derartige Konstrukte nicht teilweise um ein Körperteil umgeschlagen werden können.

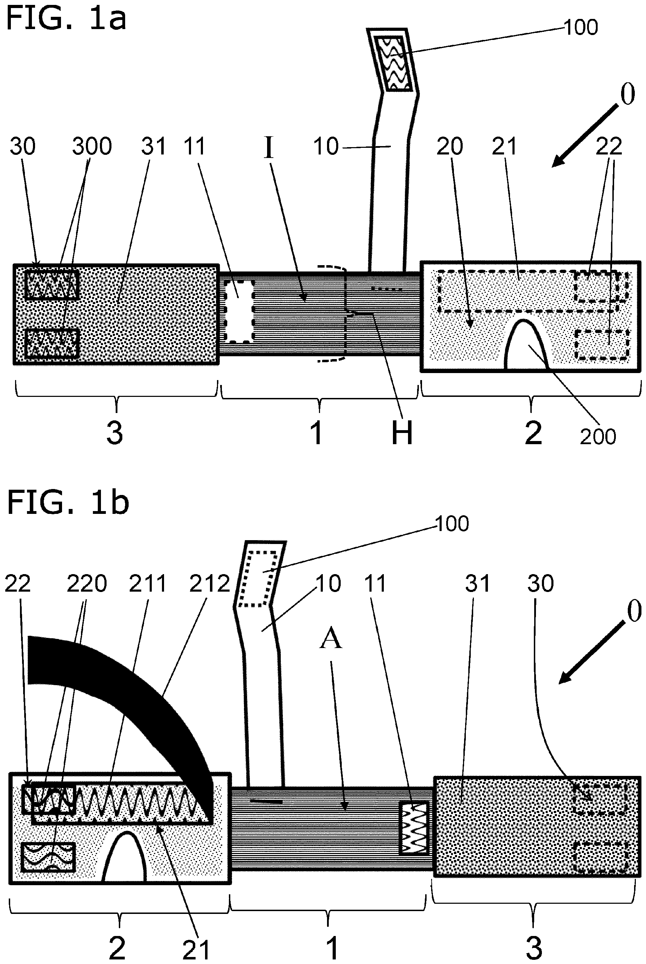

Figur 1a zeigt eine Innenfläche I eines Anästhesieschutzbandes 0, umfassend einen Zentralauflageabschnitt 1, einen auf einer ersten Seite daran angrenzenden ersten Schutzabschnitt 2 und einen auf der gegenüberliegenden Seite an den Zentralauflageabschnitt 1 angrenzenden zweiten Schutzabschnitt 3. Diese drei Abschnitte 1, 2, 3 sind miteinander unlösbar verbunden und sind aus Textilien, welche gewebt oder gewirkt sind, gebildet. Die Innenfläche I des Anästhesieschutzbandes 0 bzw. Der Abschnitte 1, 2, 3 ist im angelegten Zustand des Anästhesieschutzbandes 0 der Patientenhaut zugewandt.

Der Zentralauflageabschnitt 1 kann einlagig oder mehrlagig ausgeführt sein, wobei der Zentralauflageabschnitt 1 dehnbar in Richtung seiner Längsausdehnung, bevorzugt elastisch dehnbar um mindestens 10% seiner Länge oder mehr sein sollte. Einsetzbare stretch-Textilien sind dem Fachmann bekannt und weisen in der Regel Anteile von Elasthan bzw. Polyurethan auf. Am Zentralauflageabschnitt 1 ist ein Befestigungsband 10, vom Anästhesieschutzband 0 wegragend unlösbar befestigt, bevorzugt angenäht, angeklebt oder angeschweisst, was mit einer gestrichelten Linie auf der Innenfläche I angedeutet ist. Das Befestigungsband 10 ist bevorzugt als Textil ausgebildet und kann ebenfalls elastisch dehnbar ausgestaltet sein. Am vom Zentralauflageabschnitt 1 entfernten Ende des Befestigungsbandes 10 sind Befestigungsmittel 100 vorgesehen, welche dort unlösbar am Befestigungsband 10 verbunden angeordnet sind und welche mit Zentralbefestigungsmitteln 11 an einer Aussenfläche A des Zentralauflageabschnittes 1 lösbar wirkverbindbar sind. Eine gleichbleibende Breite H des Zentralauflageabschnittes 1 kann geringer gewählt sein, als die Breite des ersten und zweiten Schutzabschnittes 2, 3. Alle drei Abschnitte 1, 2, 3 sind voneinander optisch unterscheidbar auf der Innenfläche I und der Aussenfläche A, sodass es bei der Verwendung nicht zu Fehlern kommen kann.

Der erste Schutzabschnitt 2 ist an den Zentralauflageabschnitt 1 an einer ersten Seite angeformt bzw. unlösbar befestigt. Dieser erste Schutzabschnitt 2 kommt im angelegten Zustand des Anästhesieschutzbandes 0 direkt auf der Haut eines Patienten zu liegen.

Hier ist der erste Schutzabschnitt 2 mindestens zweilagig ausgebildet, wie beschrieben aus Textillagen gebildet, sodass in einem Innenraum zwischen den Textillagen ein oder mehrere Schutzpolster 20 eingeschoben gelagert sind. Damit die Schutzpolster 20 nicht verrutschen können, sind diese angeklebt, angenäht oder angeschweisst. Das Schutzpolster 20 oder mehrere integrierte Schutzpolster 20 sind hier so geformt, dass ein Bereich, in welchem später die Nase des Patienten zu liegen kommt, frei von einem Schutzpolster 20 ist. Es ist hier eine Nasenbereichsaussparung 200 mit weniger Polsterwirkung ausgestaltet.

Die Innenfläche I des ersten Schutzabschnittes 2 ist ansonsten möglichst glatt und weich ausgestaltet, da diese Innenfläche I beispielsweise auf dem Gesicht eines Patienten zu liegen kommt. In Figur 1a sind Schlauchbefestigungsmittel 21 und Bandverschlussmittel 22 an der Aussenfläche A des ersten Schutzabschnittes 2 angedeutet.

Auf der, dem ersten Schutzabschnitt 2 gegenüberliegenden Seite des Zentralauflageabschnittes 1 ist der zweite Schutzabschnitt 3 unlösbar verbunden bzw. angeformt. Auch der zweite Schutzabschnitt 3 ist aus mindestens einer Textillage gebildet und weist auf seiner Innenfläche I zweite Bandverschlussmittel 30 auf, welche mit den ersten Bandverschlussmitteln 22 am ersten Schutzabschnitt 2 wirkverbindbar sind. Die zweiten Bandverschlussmittel 30 sind bevorzugt als Hakenband oder Flauschband 300 ausgebildet, sodass sie mit ihren Gegenstücken einen Klettverschluss bilden.

Optional ist hier eine Polsterlage 31 an oder im zweiten Schutzabschnitt 3 angeordnet. Wenn der zweite Schutzabschnitt 3 zwei Textillagen umfasst, kann die Polsterlage 31 wiederum zwischen die Textillagen geschoben und dort befestigt werden, mittels Kleben, Nähen oder Schweissen. Dies ist aber nicht unbedingt nötig, wenn bereits der erste Schutzabschnitt 2 eine Polsterung aufweist. Die Innenfläche I des zweiten Schutzabschnittes 3 kommt nach dem Umschlagen auf der Aussenfläche A des ersten Schutzabschnittes 2 zu liegen und erste und zweite Bandverschlussmittel 22, 30 sind wirkverbindbar.

Gut erkennbar sind die Schlauchbefestigungsmittel 21, umfassend Hakenband 211 und Flauschband 212, welche wiederum einen Klettverschluss bilden, mit welchem Versorgungsschläuche und Kabel 4, wie unten eingeführt gesichert halten können. Die Wahl welches Schlauchbefestigungsmittel 21 als Hakenband 211 oder als Flauschband 212 ausgeführt ist, ist auswechselbar. Die jeweiligen Längen müssen ausreichen land gewählt sein, damit die Versorgungsschläuche und Kabel 4 korrekt platziert und befestigt werden können.

Die Naht auf der Aussenfläche A des Zentralauflageabschnittes 1 zur Befestigung des Befestigungsbandes 10 zeigt eine Möglichkeit zur Befestigung des Befestigungsbandes 10. Das Zentralbefestigungsmittel 11 ist nach Umschlagen des Befestigungsbandes 10 mit dem Befestigungsmittel 100 des Befestigungsbandes 10 wirkverbindbar bzw. das Befestigungsband 10 lösbar verbindbar. Auch hier bietet sich die Ausführung eines Klettverschlusses mit Flausch- und Hakenband für Zentralbefestigungsmittel 11 und Befestigungsmittel 100 des Befestigungsbandes 10 an.

Hier nicht dargestellt sind erster Schutzabschnitt 2 und zweiter Schutzabschnitt 3 mehrlagig und öffenbar ausgestaltet, sodass Schutzpolster 20 und Polsterlage 31 seitwärts eingeführt und entfernt werden können. Dies kann zeitlich kurz vor der Verwendung passieren. Schutzpolster 20 und Polsterlage 31 können aber auch eingeführt und unlösbar befestigt sein. Die Breiten des ersten und zweiten Schutzabschnittes 2, 3 sollten so gewählt sein, dass beispielsweise ein Gesicht eines Patienten nur partial verdeckt wird und der Mundbereich unverdeckt bleiben kann.

Das Anästhesieschutzband 0 wird wie folgt angelegt. Der Kopf K eines Patienten wird derart auf der Innenfläche I des Zentralauflageabschnitts 1 abgelegt, dass links und rechts vom Kopf K beabstandet, erster Schutzabschnitt 2 und zweiter Schutzabschnitt 3 zu liegen kommen. Erster und zweiter Schutzabschnitt 2, 3 sollen sich auf Höhe des Ausgen- und Nasenbereichs N oder des Gesichtsfeldes des Patienten befinden. Das Befestigungsband 10 ragt entsprechend vom Zentralauflageabschnitt 1 und damit vom Kopf K weg. Die Versorgungsschläuche und Kabel 4 sind noch nicht am Patienten befestigt, der Patient aber in der Regel bereits narkotisiert. Die Intubation erfolgt in der Regel während des Anlegens des Anästhesieschutzbandes 0.

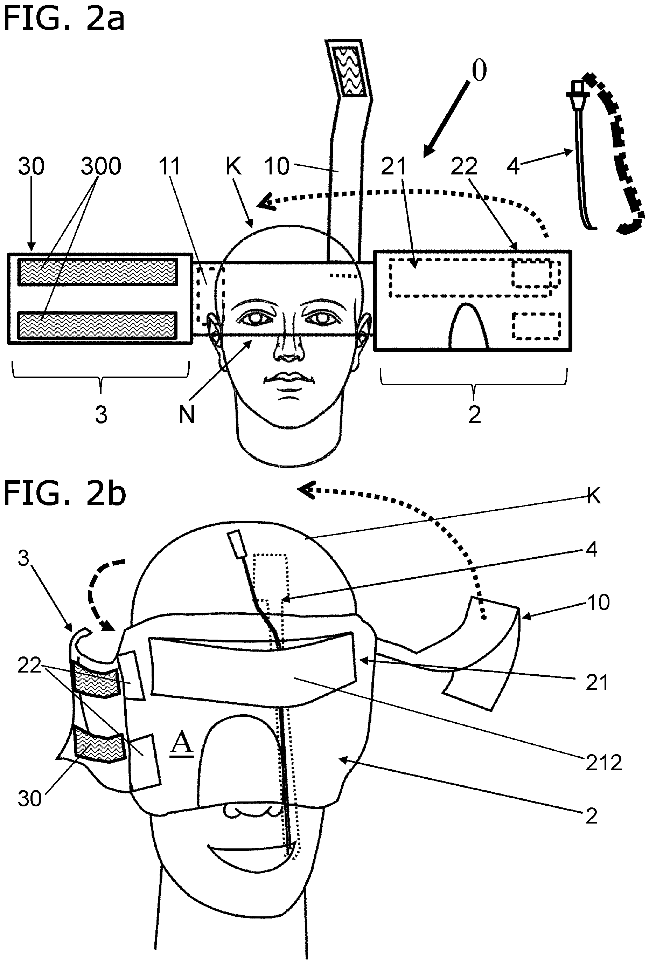

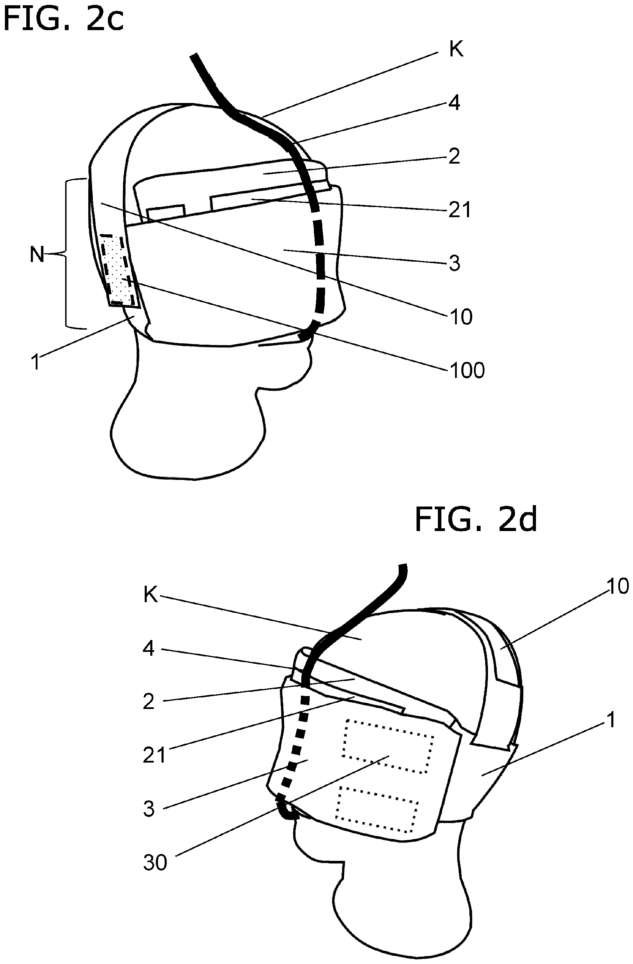

Nun wird der erste Schutzabschnitt 2 um den Kopf K geschlagen, wie mit dem gestrichelten Pfeil in Figur 2a angedeutet und über das Gesichtsfeld des Patienten gelegt, wobei die Aussenfläche des ersten Schutzabschnittes 2 vom Gesicht wegragt. Der Mundbereich des Kopfes K bleibt immer ausgespart, während die Nase noch mindestens teilweise bedeckt ist. Hier ist die Nasenbereichsaussparung 200, als Bereich ohne Schutzpolster 20 angedeutet, wobei das Schutzpolster 20 gesamthaft nicht dargestellt ist. Die ersten Bandverschlussmittel 22 am ersten Schutzabschnitt 2 verlaufen seitlich am Kopf K entlang, wobei die Innenfläche I des ersten Schutzabschnitts 2 direkt auf der Haut des Patienten liegt.

Nun werden die Versorgungsschläuche und Kabel 4 am Patienten installiert und quer über den ersten Schutzabschnitt 2 gelegt und mittels Schlauchbefestigungsmitteln 21 an dessen Aussenfläche A lösbar befestigt. Die Versorgungsschläuche und Kabel 4 werden dann vom Patienten weggeführt und mit entsprechenden Apparaturen verbunden.

Wie mit dem kurzen gestrichelten Pfeil in Figur 2b angedeutet, wird im nächsten Schritt der zweite Schutzabschnitt 3 in Frontalansicht von links nach rechts um den Kopf K geschlagen, wobei der zweite Schutzabschnitt 3 den ersten Schutzabschnitt 2 mindestens teilweise überdeckt. Die zweiten Bandverschlussmittel 30 werden dabei mit den ersten Bandverschlussmitteln 22 am ersten Schutzabschnitt 2 lösbar verbunden.

Zuletzt wird das Befestigungsband 10 über den Kopf K geschlagen, wie hier mit dem gepunkteten Pfeil angedeutet. Mit den Befestigungsmitteln 100 wird das Befestigungsband 10 an der Innenfläche I oder Aussenfläche A des Zentralauflageabschnittes 1 befestigt.

Wie in Figur 2c gezeigt, bietet es sich an die Zentralbefestigungsmittel 11 an der Aussenfläche A des Zentralauflageabschnittes 1 lösbar zu befestigen. Auch hier ist wieder ein Klettverschluss ausgebildet, wobei eine Seite Flauschband und die andere Seite ein Hakenband umfasst.

Durch den ersten Schutzabschnitt 2 mit Schutzpolster 20 und den zweiten Schutzabschnitt 3 mit oder ohne Polsterlage 31 ist der Augen- und Nasenbereich N geschützt, sodass ein Operateur sogar die Finger oder eine Hand zur Fixierung gefahrlos auf dem Augen- und Nasenbereich ablegen kann, während die Versorgungsschläuche und Kabel 4 teilweise verdeckt fixiert befestigt sind. Nur die textile Innenfläche I des Zentralauflageabschnittes 1 und des ersten Schutzabschnitts 2 liegt direkt auf der Haut des Patienten auf. Da das mindestens eine Schutzpolster 20 von textilen Lagen verdeckt ist, liegt auch das Schutzpolster 20 selbst nicht direkt auf der Haut des Patienten auf. Im Augen- und Nasenbereich N liegen erster Schutzabschnitt 2 und zweiter Schutzabschnitt 3 mehrlagig übereinander, wodurch die Schutzwirkung erhöht ist, aber niemals Schutzpolster 20 oder Polsterlage 31 direkt auf der Haut aufliegen.

Die ersten Bandverschlussmittel 22, zweiten Bandverschlussmittel 30 und die Befestigungsmittel 100 des Befestigungsbandes 10 sorgen dafür, dass das Anästhesieschutzband 0 nicht verlierbar ist und nicht verrutschen kann. Die Versorgungsschläuche und Kabel 4 werden von den Schlauchbefestigungsmitteln 21 und durch den zweiten Schutzabschnitt 3 geführt gehalten, sodass sich diese nicht lösen können und beispielsweise keine Druckstellen am Patienten erzeugen können.

Das hier beschriebene Anästhesieschutzband 0 kann, neben Schilddrüsenoperationen auch für andere operative Eingriffe am Kopf K, Hals oder auch im Bereich der Extremitäten eingesetzt werden, wobei entsprechend ein anderes Körperteil vom Zentralauflageabschnitt 1, erstem Schutzabschnitt 2 und zweitem Schutzabschnitt 3 umgeben wird, sodass die Schutzpolster 20 und/oder die Polsterlage 31 das Körperteil umgeben.

Durch Wahl gewirkter und/oder gestrickter Textilien für Zentralauflageabschnitt 1, ersten Schutzabschnitt 2 und zweiten Schutzabschnitt 3, ist eine gewünschte Atmungsaktivität erreicht, sodass feuchtwarme Luft von der Haut des Patienten entweichen kann. Bevorzugt wird ein Gestrick, insbesondere ein Jersey oder Trikotstoff verwendet. Im besten Fall sind sämtliche Abschnitte 1, 2, 3 aus einem weichen, elastischen Stoff gebildet. Das Gestrick ist aus Viskose oder Viskosemischungen, Kunstfasern im Allgemeinen, Wollmischgarnen oder Baumwolle herstellbar, welches atmungsaktiv und saugfähig ist.

Geeignete Materialien für das Garn der Bestandteile des Anästhesieschutzbandes 0 sind bekannt und können Natur- und/oder Kunststofffasern umfassen. Bevorzugt sind die Garne aus hypoallergenen Materialien gebildet. Das Material sollte frei von Duft-, Farb- und Konservierungsstoffen sein, also frei von Stoffen mit hohem allergologischen Risiko. Das Garn des mit der Haut in Berührung kommenden ersten Schutzabschnittes 2 sollte kontaktallergenfrei ausgebildet sein. Zusätzlich kann eine antibakterielle und/oder antimikrobielle Beschichtung aufgebracht sein kann.

Bevorzugt werden erster Schutzabschnitt 2 und zweiter Schutzabschnitt 3 aus einem Flachstrick oder Schlauchware konfektioniert, wobei die Schutzpolster 20 und/oder die Polsterlage 31 Einlagen aus PE-Vlies oder PU-Schaum sind.

Das Schutzpolster 20 bzw. die Polsterlage 31, wenn vorhanden, sollte eine Dicke von mindestens einem Zentimeter, bevorzugt mindestens zwei Zentimetern aufweisen. Die Schutzpolster 20 können einstückig aus einem Material gefertigt sein. Schaummaterialien bieten sich als Schutzpolster 20 an, welche jeweils beidseitig von den Textillagen des ersten Schutzabschnittes 2 umhüllt sind.

Das Material für die Befestigungsmittel 100, Schlauchebefestigungsmittel 21, erste Bandverschlussmittel 22 und/oder zweite Bandverschlussmittel 30 bzw. die Klettverschlüsse ist aus Polyamid-, Polyester-, Polyaramid- und/oder Polyolefinfasern, meist gewebt hergestellt. Bei den Hakenbändern sind die Haken während des Webens oder später eingearbeitet. Hier sind mit Klettverschluss alle möglichen Arten von Haken- und Flauschverschlüssen gemeint, welche bereits seit längerem in der Textilindustrie eingesetzt werden.

Die Befestigungsmittel 100, Schlauchebefestigungsmittel 21, erste Bandverschlussmittel 22 und/oder zweite Bandverschlussmittel 30 sind aus Micro-Klett und auf die jeweiligen Textilien aufgenäht, wobei auch sogenannter elastischer Klett verwendet werden kann.

### Bezugszeichenliste

0 Anästhesieschutzband vorrichtung=Gesichtsband, teilweise mehrlagig, teilweise gepolstertes Textil
I Innenfläche
A Aussenfläche
K Kopf eines Patienten
N Augen- und Nasenbereich
1 Zentralauflageabschnitt
   10 Befestigungsband (Überkopfbefestigungsband)
   100 Befestigungsmittel des Befestigungsbandes an dessen Innenfläche
   11 Zentralbefestigungsmittel an Aussenfläche A für lösbare Befestigung des Überkopfbefestigungsbandes
   12 Überkopfbefestigungsbandbefestigungsmittel H Breite
2 erster Schutzabschnitt
   20 Schutzpolster
      200 Nasenbereichsaussparung (ohne Polster oder weniger Polster)
   21 Schlauchbefestigungsmittel an dessen Aussenfläche für Schläuche und Kabel
      211 Hakenband
      212 Flauschband
   22 erste Bandverschlussmittel am ersten Schutzabschnitt
      220 Hakenband oder Flauschband
3 Zweiter Schutzabschnitt (optional gepolstert)
   30 zweite Bandverschlussmittel
   300 Hakenband oder Flauschband
   31 Polsterlage
4 Versorgungsschläuche und Kabel

## Patentansprüche

1. Anästhesieschutzvorrichtung (0) für einen Patienten, welche vor einem operativen Eingriff appliziert wird, wobei die Anästhesieschutzvorrichtung (0) von einem einstückigen teilweise mehrlagigen und teilweise gepolsterten Textil (0) mit einer Innenfläche (I) gebildet ist, wobei die Anästhesieschutzvorrichtung (0) derart um einen Körperteil des Patienten derart legbar ist, die Innenfläche (I) mindestens teilweise direkt auf der Haut des Patienten aufliegt und die Anästhesieschutzvorrichtung (0) lösbar am Patienten befestigbar ist, **dadurch gekennzeichnet, dass**
die Anästhesieschutzvorrichtung (0) als offenes, teilweise mehrlagiges und gepolstert ausgeführtes textiles Anästhesieschutzband (0) ausgestaltet ist, umfassend einen Zentralauflageabschnitt (1), an welchem an einer ersten Seite ein erster Schutzabschnitt (2) mit mindestens einem Schutzpolster (20) zwischen zwei textilen Lagen des ersten Schutzabschnitts (2) eingeführt, mit Schlauchbefestigungsmitteln (21) und ersten Bandverschlussmitteln (22) auf der Aussenfläche (A) des ersten Schutzabschnittes (2) aufweisend, angeformt ist,
sowie an einer der ersten Seite gegenüberliegenden zweiten Seite des Zentralauflageabschnitts (1)
ein zweiter Schutzabschnitt (3) aus einem mindestens einlagigem Textil mit zweiten Bandverschlussmitteln (30) an der Innenfläche (I) des zweiten Schutzabschnitts (3) unlösbar befestigt ist, sodass nach dem Umschlagen des Anästhesieschutzbandes (0) um einen Körperteil eine lösbare Verbindung mittels erster Bandverschlussmittel (22) und zweiter Bandverschlussmittel (30) ausbildbar ist.

2. Anästhesieschutzvorrichtung (0) nach Anspruch 1, wobei das mindestens eine Schutzpolster (20) im Innenraum zwischen den zwei textilen Lagen des ersten Schutzabschnittes (2) unlösbar mittels Kleben, Nähen oder Schweissen befestigt ist.

3. Anästhesieschutzvorrichtung (0) nach Anspruch 1, wobei der Innenraum zwischen den zwei textilen Lagen des ersten Schutzabschnittes (2) zugänglich ist und ein Schutzpolster (20) einführbar und nach Benutzung wieder entfernbar ist.

4. Anästhesieschutzvorrichtung (0) nach einem der vorhergehenden Ansprüche, wobei die Schlauchbefestigungsmittel (21) und die ersten Bandverschlussmittel (22) an der Aussenfläche (A) des ersten Schutzabschnittes (2) und die zweiten Bandverschlussmittel (30) an der Innenfläche (I) des zweiten Schutzabschnittes (3) unlösbar durch Kleben, Nähen oder Schweissen befestigt sind.

5. Anästhesieschutzvorrichtung (0) nach einem der vorhergehenden Ansprüche, wobei am Zentralauflageabschnitt (1) ein Befestigungsband (10) als Textil ausgebildet, Befestigungsmittel (100) aufweisend,
von der Längsrichtung des Anästhesieschutzbandes (0) wegragend verlaufend und
ein Zentralbefestigungsmittel (11)
an der Aussenfläche (A) des Zentralauflageabschnittes (1) unlösbar befestigt,
angeordnet sind.

6. Anästhesieschutzvorrichtung (0) nach einem der vorhergehenden Ansprüche, wobei eine Breite (H) des Zentralabschnittes (1) kleiner ausgeführt ist, als die Breiten des ersten und zweiten Schutzabschnittes (2, 3).

7. Anästhesieschutzvorrichtung (0) nach einem der vorhergehenden Ansprüche, wobei gewirkte und/oder gestrickte Textilien für Zentralauflageabschnitt (1), ersten Schutzabschnitt (2) und zweiten Schutzabschnitt (3) gewählt sind.

8. Anästhesieschutzvorrichtung (0) nach einem der vorhergehenden Ansprüche, wobei der Zentralauflageabschnitt (1) von einem elastisch dehnbaren Textil auf Basis von Elasthan bzw. Polyurethan gebildet ist, wobei der Zentralauflageabschnitt (1) bevorzugt in Richtung seiner Längsausdehnung um mindestens 10% seiner Länge elastisch dehnbar ist.

9. Anästhesieschutzvorrichtung (0) nach einem der vorhergehenden Ansprüche, wobei der zweite Schutzabschnitt (3) von zwei Textillagen gebildet ist und in seinem Innenraum eine Polsterlage (31) eingelegt und fixiert ist.

10. Anästhesieschutzvorrichtung (0) nach Anspruch x-1, wobei der zweite Schutzabschnitt (3) seitlich offen ausgeführt ist, sodass der Innenraum des zweiten Schutzabschnitts (3) zugänglich bleibt.

11. Anästhesieschutzvorrichtung (0) nach einem der vorhergehenden Ansprüche, wobei Zentralbefestigungsmittel (11) und Befestigungsmittel (100), Schlauchbefestigungsmittel (21), erste Bandverschlussmittel (22) und zweite Bandverschlussmittel (30) als Klettverschlüsse mit Hakenband und Flauschband ausgestaltet sind.

12. Anästhesieschutzvorrichtung (0) nach einem der vorhergehenden Ansprüche, wobei die Textilien durch kontaktallergenfreies Garn ausgebildet sind bzw. das Garn eine antibakterielle und/oder antimikrobielle Beschichtung aufweist.

13. Anästhesieschutzvorrichtung (0) nach einem der vorhergehenden Ansprüche, wobei das mindestens eine Schutzpolster (20) eine Dicke von mindestens einem Zentimeter, bevorzugt mindestens zwei Zentimetern aufweist.

14. Verfahren zum Anlegen eines mehrlagigen, teilweise gepolsterten textilen Anästhesieschutzbandes (0),
**dadurch gekennzeichnet, dass**
ein offenes, teilweise mehrlagiges und gepolstert ausgeführtes textiles Anästhesieschutzband (0), umfassend einen Zentralauflageabschnitt (1), an welchem an einer ersten Seite ein erster Schutzabschnitt (2) mindestens ein Schutzpolster (20), Schlauchbefestigungsmittel (21) und erste Bandverschlussmittel (22) aufweisend, sowie an einer der ersten Seite gegenüberliegenden zweiten Seite des Zentralauflageabschnitts (1) ein zweiter Schutzabschnitt (3) aus einem mindestens einlagigem Textil mit zweiten Bandverschlussmitteln (30) unlösbar befestigt ist, verwendet wird,
das Anästhesieschutzband (0) flach auf einer Unterlage ausgelegt wird,
ein Körperteil eines Patienten auf den Zentralauflageabschnitt (1) platziert wird, bevor der erste Schutzabschnitt (2) um das Körperteil umgeschlagen wird,
Versorgungsschläuche und Kabel (4) mittels ersten Bandverschlussmitteln (22) an der Aussenfläche (A) des ersten Schutzabschnitts (2) geführt befestigt werden, bevor der zweite Schutzabschnitt (3) um das Körperteil und den ersten Schutzabschnitt (2) geschlagen wird und zweite Bandverschlussmittel (30) mit ersten Bandverschlussmitteln (22) wirkverbunden werden.

15. Verfahren nach Anspruch 14, wobei das Anästhesieschutzband (0) mittels Befetigungsband (10) am Zentralauflageabschnitt (1) durch Wirkverbindung der Befestigungsmittel (100) mit Zentralbefestigungsmitteln (11) zusätzlich gesichert fixiert wird.
